# EUROPEAN PATENT APPLICATION

(11) **EP 2 957 569 A1**
(43) Date of publication of application: **23.12.2015**
(21) Application number: 13837467.3
(22) Date of filing: 13.09.2013
(51) Int. Cl.: C07K 7/14, A61K 38/08, A61P 9/08

(54) **(ARGO)N-ANGIOTENSIN-(1-7) PEPTIDE AND PHARMACEUTICAL COMPOSITIONS FOR TREATING DISEASES**

(30) Priority: 14.09.2012 BR 102012023206; 11.09.2013 BR 102013023224
(71) Applicant: Universidade Federal De Minas Gerais - UFMG, 31270-901 Belo Horizonte MG (BR)
(72) Inventor: SOUZA DOS SANTOS, Robson Augusto, CEP-31310-340 Belo Horizonte - Minas Gerais (BR); FERNANDES VILAS BÔAS, Suéllen Kathiane, CEP: 30.330-020 Belo Horizonte - Minas Gerais (BR); FÉLIX BRAGA, Janaina, CEP: 30.850-700 Belo Horizonte - Minas Gerais (BR); JEAN GEORGES FREZARD, Frederic, CEP: 30380-232 Belo Horizonte - Minas Gerais (BR); DARIO SINISTERRA, Ruben, CEP: 31310-390 Belo Horizonte - Minas Gerais (BR); CALDEIRA SILVA, Neiva, CEP: 30570-640 Belo Horizonte - Minas Gerais (BR); QUEIROGA LAUTNER, Roberto, CEP: 31160-190 Belo Horizonte - Minas Gerais (BR); ARAÚJO FRAGA DA SILVA, Rodrigo, CEP: 34000-000 Nova Lima - Minas Gerais (BR)
(74) Representative: Chapman, Paul Gilmour
(86) International application number: PCT/BR2013/000362
(87) International publication number: WO 2014/040158

(57) **Abstract**

The present invention relates to the peptide (arg0) n-angiotensin-(1-7) [(arg0) n-Ang-(1-7)], where n is 1 to 10 (Xaa-Asp-Arg-Val-Tyr-Ile-His-Pro; SEQ ID NO:1 where Xaa represents 1 to 10 L-Arg residues), which is produced by inserting at least one arginine amino acid at the amino terminal position of Ang-(1-7), as well as pharmaceutical compositions containing this peptide and the use thereof for the treatment or prevention of diseases or disorders that are due or associated with reduced nitric oxide production. Non-limiting examples of these diseases or disorders are cardiopulmonary and liver diseases, vascular disorders, metabolic disorders, neural disorders, genito-urinary tract disorders, skeletal muscle disorders, kidney disorders, skin disorders, alopecia or tumors. The peptide is able to cause, for example, dilation of aortic and mesenteric vessels, reduction of mean arterial blood pressure, decrease of body weight gain, blockade of the development of visceral adiposity, reduced serum and liver cholesterol levels, the normalization of glucose intolerance and insulin resistance, and an anti-proliferative effect. The peptides of the invention, such as (arg0) n-Ang-(1-7), optionally can be associated with a carrier system or controlled drug release system.

## Description

### DISCUSSION

The present invention relates to the peptide (arg0) n-angiotensin- (1-7) [(arg0) n-Ang-(1-7)], where n is 1 to 10 (Xaa-Asp-Arg-Val-Tyr-Ile-His-Pro; SEQ ID NO:1 where Xaa represents Argₙ-, where n = 1-10) which is produced by inserting at least one amino acid arginine in the amino terminal position of Ang-(1-7), as well as pharmaceutical compositions containing this peptide and the use thereof for the treatment or prevention of diseases or disorders that are due to or associated with reduced nitric oxide production, and as non-limiting examples cardiopulmonary and liver diseases, vascular disorders, metabolic disorders, neural disorders, disorders of the genito-urinary tract, skeletal muscle, kidney, or skin, and alopecia or tumors. The peptide is able to cause, for example, dilation of aortic and mesenteric vessels, reduction of mean arterial blood pressure, decrease of body weight gain, blockade of the development of visceral adiposity, reduction of serum and liver cholesterol levels, normalization of glucose intolerance and insulin resistance, and an anti-proliferative effect. The peptide (arg0) n-Ang-(1-7) (SEQ ID NO:1) can be optionally associated with a carrier system or controlled drug release system.

The peptide, subject of the present invention, is obtained based on the peptide angiotensin (1-7), one of the molecules involved in the renin-angiotensin system (RAS). The RAS is a hormone, circulating and local system responsible for regulating renal and cardiovascular functions by exerting a central role in the homeostasis of blood pressure and electrolyte balance.

Classically, activation of RAS begins with the enzyme renin, which is synthesized and released by the juxtaglomerular cells of the renal afferent arterioles. This enzyme, in turn, cleaves angiotensinogen (AGT), forming an inactive decapeptide, angiotensin I (Ang I) (Fyhrquist, F. & Saijonmaaa, O. et al. Renin-Angiotensin System Revisited. Journal of Internal Medicine, vol. 3, p. 224-236, 2008). The Angiotensin Converting Enzyme (ACE), another component of the RAS is an enzyme present on the luminal surface of the vascular endothelium containing two active domains which are inhibited by ACE inhibitors. It is responsible for catalyzing the removal of two amino acids of the amino-terminal portion of Ang I, yielding a biologically active octapeptide, Ang II. Moreover, this enzyme also hydrolyzes bradykinin and the heptapeptide Ang-(1-7) to inactive metabolites (Turner, A. et al. The Angiotensin-Converting Enzyme Gene Family: Genomics and Pharmacology Trends in Pharmacological Sciences, v.23, n. 4, p. 177-183, 2002). Thus, ACE participates in the cardiovascular control through degradation of vasodilatory peptides and by contributing to the formation of Ang II (Wei, C. et al. Differential ANG II Generation in Plasma of Mice and Tissue with Decreased Expression of the ACE gene. American Journal of Physiology. Heart and Circulatory Physiology, v. 282, n. 6, p. H2254-2258, 2002).

Both Ang I and Ang II can be hydrolyzed by ACE2, forming Ang-(1-7), a biologically active component of RAS. ACE2 is a homologous enzyme of ACE, but it is not inhibited by the angiotensin-converting enzyme inhibitors. However, the catalytic activity of ACE2 in hydrolyzing Ang II is 400 times higher than that for Ang I. To hydrolyze Ang I this enzyme (ACE2), removes a single C-terminal residue from Ang I to form a peptide angiotensin-(1- 9) [(Ang-(1-9)]. This can be converted to Ang-(1-7) by the action of ACE and other endopeptidases (Donoghue M. et al., A Novel Angiotensin-Converting Enzyme-Related Carboxypeptidase (ACE2) Converts Angiotensin I to Angiotensin 1-9 Circulation Research, vol 87, p E1-E9, 2000; Santos, RAS et al Recents Advances in the Angiotensin-Converting Enzyme 2-Angiotensin- (1-7). -MAS Axis. Experimental Physiology, vol. 5, p. 519-527, 2008).

Additional peptides of the family of angiotensin include angiotensin III and angiotensin IV (Ang IV), which are less characterized. These peptides antagonize or have additional effects compared to Ang II (Fyhrquist, F. & Saijonmaaa, O. et al. Renin-Angiotensin System Revisited. Journal of Internal Medicine, vol. 3, p. 224-236, 2008).

Ang-(1-7) has often opposite actions to those attributed to Ang II, such as vasodilation mediated by nitric oxide and prostaglandins, antithrombotic, anti-arrhythmogenic, anti-proliferative, anti-tumorigenic, cardio protective and metabolic protective effects, inhibition of norepinephrine release, modulation of tonic control and reflex of blood pressure, increasing of the sensitivity of the baroreceptor reflex, activation of the insulin signaling pathway by increasing the phosphorylation of AKT, prevention of skeletal muscular dystrophy, and effects on the processes of ovulation and spermatogenesis. Thus, the heptapeptide has been considered the main regulator of the counter-deleterious effects of Ang II (Marques, F. et al. An Oral Formulation of Angiotensin-(1-7) produces cardioprotective effects in infarcted and isoproterenol-treated rats. Hypertension v 3, p 477-483, 2011; Santos, R et al. Recent Advances in the Angiotensin-Converting Enzyme 2-Angiotensin- (1-7)-MAS Axis Experimental Physiology, vol. 5, p 519-527, 2008; Ribeiro-Oliveira, A Jr. et al. The Renin Angiotensin System-and Diabetes: an update vascular health and risk management, v.4, p 787-803, 2008; Santos, R et al. Angiotensin-(1-7): an update Regulatory Peptides, vol. 1-3, p 45-62, 2000).

Recently, the receptor coupled to protein G Mas (GPCR) has been identified as a receptor for Ang-(1-7). It has been reported in the art that the biologically active fragment angiotensinergic, Ang-(1-7) (Asp-Arg-Val-Tyr-Ile-His-Pro; SEQ ID NO:2), is a ligand with high affinity for the receptor Mas. This receptor is involved in several actions of Ang-(1-7), including the regulation of blood pressure and improving metabolic, and anti-thrombotic and anti-carcinogenic effects (Santos, R. et al. Angiotensin-(1-7) is an endogenous ligand for the G protein-coupled receptor MAS. Proceedings of the National Academy of Science, vol. 100, n 14, p 8258-8263, 2003; Santos, R et al. Recents Advances in the Angiotensin-Converting Enzyme 2-Angiotensin-(1-7)-MAS Axis. Experimental Physiology, Vol. 5, p. 519-527, 2008).

Several actions of Ang-(1-7) are blocked by a selective receptor antagonist of Mas, A-779 (Asp-Arg-Val-Tyr-Ile-His-D-Ala; SEQ ID NO:3), although some effects of Ang-(1-7) are only partially blocked by it. Moreover, the literature reports that some of the actions of the heptapeptide may involve ACE, AT1 and AT2 receptors. More recent studies, have shown the possible existence of a different receptor subtype for Ang-(1-7) in aorta of Sprague-Dawley rats sensitive to another receptor antagonist, Mas, D-Pro7-Ang-(1-7) (Asp-Arg-Val-Tyr-Ile-His-D-Pro; SEQ ID NO:4) (Silva, DMR et al. Evidence for a new angiotensin-(1-7) receptor subtype in the aorta of Sprague-Dawley rats. Peptides, vol. 28, n. 3, p. 702-707, 2007).

The cardiovascular and baroreflexive actions of Ang-(1-7) counterregulate the deleterious effects of Ang II. For example, Ang II, acting via the receptor AT1 causes vasoconstriction and concomitant increases blood pressure, while Ang-(1-7), acting via Mas receptor, causes vasodilation and decreases pressure. The Ang-(1-7) has a vasodilatation effect on several vascular beds, including canine and porcine coronary artery, rat aorta and mammal mesenteric artery. Furthermore, it was shown that Ang-(1-7) blocked the vasoconstriction induced by Ang II in isolated human arteries and forearm circulation in normotensive men. It is believed that the vasodilatory effects of bradykinin are potentiated by Ang-(1-7) (Santos, R, et al. Angiotensin-(1-7): An update Regulatory Peptides, vol 1-3, 45- 62, 2000).

The vasodilatory effect of heptapeptide via Mas occurs by the release of nitric oxide (NO). The Ang-(1-7) stimulates the activation of endothelial nitric oxide synthase (eNOS) and, consequently, the production of NO via Akt dependent signaling (Sampaio, Wo et al. Angiotensin- (1-7) receptor through MAS endothelial nitric oxide synthase mediates activation Akt-dependent pathways. Hypertension, vol. 49, n. 1, p. 185-192, 2007).

NO is produced by many cells throughout the body and is responsible for the production of nitric oxide synthase, which is capable of generating NO and L-citruline from L-arginine. The presence of nitric oxide within the vascular endothelium is particularly important for the regulation of blood flow, and its abnormal production, as in various disease states, may affect blood flow and other functions.

In addition to its actions in the vascular system, NO participates in many physiological processes in the central and peripheral nervous system, preventing the development of stroke, in the control of infections and malignancies, in the regulation of renal microcirculation and as a mediator of penile erection (Moncada, S. et al., The Discovery of Nitric Oxide and its Role in Vascular Biology. British Journal of Pharmacology, vol. 147, suppl. 1, pp. S193-S201, 2006).

NO has been identified as a mediator in inhibitory non-adrenergic, non-cholinergic (NANC) neurotransmission in peripheral nerves. This 'nitrergic' neurotransmission has been demonstrated in the corpus cavernosum, resulting in erection of the penis. Thus, selective nitrergic nerve damage can lead to erectile dysfunction (Moncada, S., et al., The Discovery of Nitric Oxide and its Role in Vascular Biology. British Journal of Pharmacology, vol. 147, suppl. 1, pp. S193-S201, 2006). Due to the vasodilatation properties of Ang-(1-7), the shaft Ang-(1-7)/Mas receptor also plays a role in penile erection. It was observed that the infusion of Ang-(1-7) in rats induced the release of NO and enhanced the elevation of pressure in the cavernous bodies. These effects were blocked by A-779 and by N-nitro-L-Arginine methylester (L-NAME), an inhibitor of the enzyme NO synthase. In addition, mice with a genetic deletion of the receptor Mas showed impairment of erectile function, as demonstrated by the penile fibrosis and decreased response to stimulation of the pelvic ganglion (Goncalves, et al. Evidence that the Angiotensin-(1-7)-Mas Axis Vasodilator plays an Important Role in Erectile Function. American Journal of Physiology. Heart and Circulation Physiology, v. 293, n. 4, p. H2588- H2596, 2007).

The RAS also has an important role in metabolic homeostasis. Clinical trials and animal models of type I and type II diabetes have shown that hyperactivity of Ang II signaling pathways contributes to the development of insulin resistance and diabetes (Velloso, LA et al. The multi-faceted cross-talk between angiotensin II and insulin signaling systems. Diabetes/Metabolism Research and Reviews, v. 22, n. 2, p. 98-107, 2006). On the other hand, the heptapeptide Ang-(1-7) exerts a protective action by increasing insulin sensitivity via the IRS-1/PI3K/AKT pathway and improving lipid metabolism (Giani, JF et al. Chronic infusion of angiotensin-(1-7) Improves hypertension and insulin resistance induced by high-fructose diet in rats American Journal of Physiology - Endocrinology and Metabolism, vol. 296, pp 262-271, 2009; Santos et al. SHS Improved glucose and lipid metabolism in transgenic rats with Increased circulating angiotensin-(1-7). Arteriosclerosis, Thrombosis, and Vascular Biology, v. 30, p. 953-61, 2010). These effects are mediated via the Mas receptor since the specific antagonist of this receptor, A-779, blocked the actions of Ang-(1-7). In addition, mice with a genetic deletion of the receptor Mas were characterized as a model of metabolic syndrome by increasing epididymal adipose tissue, insulin resistance, and dyslipidemia (Santos, et al. SHS. But Deficiency in FVB/N Mice Produce Marked Changes in Lipid Glycemic and Metabolism. Diabetes, vol. 57, p. 340-347, 2008).

It has been described that there is an inverse correlation between the metastatic potential of tumors and production of NO in the development of tumor vascularization, and potential injury is related to low concentrations of NO. The anti-tumorigenic NO actions are related to the activation of nuclear factor P-53, a transcription factor involved in apoptosis of abnormal or damaged cells and the activation of the inducible isoform of the enzyme NO synthase. (Muntane, J.; De La Mata, M. Nitric Oxide and Cancer. World Journal of Hepatology, v. 9, n. 2, p. 337-344, 2010). The shaft Ang-(1-7)/Mas receptor also has an important anti-tumorigenic and anti-proliferative effect. Verano-Braga et al. showed that human endothelial cells and A549 cells (human alveolar adenocarcinoma), when stimulated with Ang-(1-7) were dephosphorylated at site S256 of the transcription factor forkhead box O one protein (FoxO1). Consequently, there was a translocation of this protein from cytoplasm to the nucleus, where it becomes active and regulates several genes involved in cell proliferation, apoptosis, DNA damage repair and tumor suppression. This suggests that the anti-carcinogenic activity of Ang(1-7) involves the activation of FoxO1, as well as the inactivation of proteins involved in cell proliferation and tumorigenesis such as MAPK1, HDAC1 and mTORC1 (Verano-Braga, T. et al. Time-resolved Quantitative Phosphoproteomics: New Insights into Angiotensin-(1-7) Signaling in Human Endothelial Cells Networks Journal of proteome research, Vol 6, No 11, pp 3370-3381, 2012).

NO plays an important role in the regulation of cerebral blood flow and protection of nerve cells against pathogenic factors associated with cerebral ischemia, trauma, and hemorrhage (Toda, N. et al. Cerebral Blood Flow Regulation by Nitric Oxide: Recent Advances Pharmacological Reviews. , v. 61, n. 1, p. 62-97, 2009). The shaft Ang-(1-7)/Mas receptor also has beneficial effects against the neurological deficits and central nervous system injury as a consequence of stroke. A study has shown that the intracerebroventricular infusion of Ang-(1-7) in a model of cerebral ischemia reduced the infarct size and neurological brain area payment deficits, and that these effects were blocked by Mas receptor inhibitor, A-779 (Meca, AP et al. Cerebroprotection by Angiotensin-(1-7) in Endothelin-1-induced ischemic Stroke. Experimental Physiology, vol. 96, n.10, p.1084-1096, 2011).

The protective effect of Ang-(1-7) in the cardiopulmonary system was demonstrated by a study in which it was observed that overexpression of the heptapeptide reduced bleomycin-induced pulmonary fibrosis, and reversed cardiopulmonary pressure in monocrotaline model. These effects were abolished by receptor antagonist Mas A-779 (Shenoy, V. et al. The Angiotensin-Converting Enzyme 2/Angiotensin-(1-7)/Mas Axis Cardiopulmonary Confers Protection Against Lung Fibrosis and Pulmonary Hypertension. American Journal of Respiratory and Critical Care Medicine, vol. 182, p. 1065-1072, 2010).

One study, not yet published, showed the potential of Ang-(1-7) as a therapeutic agent for the treatment of skeletal muscle dystrophy both in vitro and in vivo, and that the beneficial effects in reducing muscle fibrosis and increasing contractile force were reversed in the presence receptor antagonist Mas, A- 779 (Acuna, MJ et al. Angiotensin (1-7), A Novel Agent That Reduces Fibrosis And Improves Muscle Strength In Dystrophic Skeletal Muscle. New Directions in Biology and Disease of Skeletal Muscle Conference. New Orleans, Louisiana, 2012).

Although both Ang-(1-7) and NO are widely known, the art describes a peptide that comprises a sequence of a precursor of NO and angiotensin-(1-7). The peptide (arg0) n-angiotensin- (1-7) [(arg0) n-Ang-(1-7); SEQ ID NO:1], of this invention contains L-arginine, a precursor of NO, and the sequence of Ang-(1-7).

Prior art patent application PI0800585-0, entitled "Peptide Des- [Asp1] - [Ala1] - agonist angiotensin (1-7) and pharmaceutical compositions for treating diseases", describes a peptide derived from Ang-(1-7), which was obtained by replacing the aspartate (Asp), the first amino acid at the N-terminal position of Ang-(1-7), with alanine (Ala) (Ala-Arg-Val-Tyr-Ile-His-Pro; SEQ ID NO:5. This heptapeptide has vascular protective and cardioprotective effect in mammals. Therefore, it is a different product object of this invention, since (arg0) n-Ang- (1-7) is an octapeptide having at least one arginine (Arg) inserted in the N-terminal position of Ang-(1-7). Furthermore, the peptide of this application PI0800585-0 has limited effects to the cardiovascular system.

The patent application GB 1487115 titled "Angiotensins antagonist" describes an octapeptide the sequence of which is R-Arg-Val-Tyr-Ile-His-Pro-X, wherein R represents N, N-dimethylglycyl, N -methylisoleucil, guanidylacetyl or sarkosyl and X is threonine, O-methyl-threonine or isoleucine (SEQ ID NO:6). This peptide is able to inhibit vasoconstrictor effects of angiotensin II. However, this is a different peptide which is the object of the present invention.

### SUMMARY OF THE INVENTION

The peptide (arg0) n-Ang-(1-7) (SEQ ID NO:1) can be used for treating or preventing diseases or cardiopulmonary disorders, vascular or renal disorders, or metabolic diseases and disorders, including, but not limiting to, hypertension, essential hypertension or secondary renal vascular hypertension, atherosclerosis, damage due to ischemia/reperfusion injury, acute myocardial infarction, acute congestive heart failure or chronic left ventricular hypertrophy, vascular hypertrophy, glaucoma, primary or secondary hyperaldosteronism, diabetic neuropathy, glomerulonephritis, scleroderma, glomerular sclerosis, renal insufficiency, organ transplant therapy, diabetic retinopathy, nephropathy, and erectile dysfunction, dyslipidemia, insulin resistance, type 1 diabetes, type 2 diabetes, or metabolic syndrome.

The peptide (arg0) n-Ang-(1-7) also can be used as cardioprotective and metabolic protective agent in patients having risk factors for diseases and/or disorders described above. Another use of the peptide is for the treatment, prevention and/or management of neoplasms, benign or malignant, not limited to a specific body region. Moreover, this peptide can be used for the treatment, prevention and/or management of neurological diseases or disorders, since AT participates in the physiological processes related to the central and peripheral nervous systems. Such diseases or disorders include, but are not limited to, diabetic peripheral neuropathy, pain, cerebral vascular accident and cerebral ischemia. The peptide (arg0) n-Ang-(1-7) also can be used as neuroprotective agents in patients having risk factors for the diseases and/or disorders described above. The peptide (arg0) n-Ang-(1-7) also can be therapeutic agent in the treatment of skeletal muscle dystrophy. The peptide (arg0) n-Ang-(1-7) also can be used in the prevention and treatment of alopecia.

These peptide derivatives and those which are t resistant to hydrolysis, such as, but not limited to, cyclic or esterified forms, also can be used for prevention and/or treatment of diseases which can be treated and/or prevented by receptor agonists Mas, as the angiotensin-(1-7), AVE0991 and CGEN 856.

The invention comprises, but is not limted to a peptide Xaa-Asp-Arg-Val-Tyr-Ile-His-Pro where Xaa represents 1 to 10 Arg amino acid residues (SEQ ID NO:1) and a pharmaceutical composition comprising this peptide and a pharmaceutically acceptable excipient. In some embodiments, the pharmaceutical composition comprises or further comprises a carrier system or controlled drug release system, such as those selected from the group consisting of a cyclodextrin, a polymer, a mucoadhesive polymer, lipidic vesicles, liposomes, polymersomes, solid lipid nanoparticles, polymeric microparticles, nanoparticles, microcapsules, nanocapsules, a microemulsion, a nanoemulsion, dendrimers, micelles, polymeric micelles, inorganic nanoparticles, carbon nanoparticles, a transdermal patch, an implantable device, and deployable polymeric matrices. The products of the invention can be configured for administration by oral, intramuscular, intravenous, subcutaneous, topical, transdermal, nasal, pulmonary, and inhalation routes, and by implantable or injectable devices, preferably for oral administration.

Other embodiments of the invention include a method of treatment of diseases or disorders of the genito-urinary tract, liver, cardiopulmonary, metabolic, neural, skeletal muscle, kidney, skin, cancer, alopecia, or vascular disorders in a subject in need thereof, by administering a peptide or pharmaceutical composition as described above.

### BRIEF DESCRIPTION OF THE FIGURES

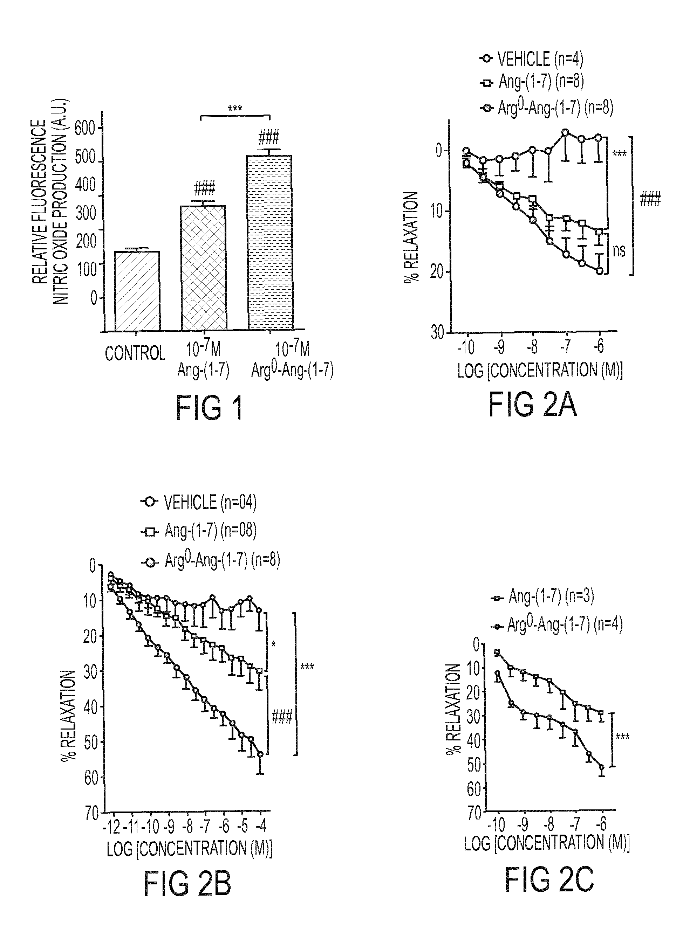

FIG. 1 shows the production of nitric oxide by the arg0-Ang-(1-7) and Ang-(1-7) peptides in Chinese hamster ovary cells in culture transfected with the Mas receptor. Each point shows the mean ± standard error of the mean. The symbol (###) indicates p <0.0001 compared to the control and the symbol (***) indicates p <0.0001 compared to Ang (1-7).

FIG. 2A and 2B show the concentration-dependent vasodilatation effects of arg0 peptide-Ang-(1-7) and Ang-(1-7) in the thoracic aortic rings of normotensive Wistar rats and spontaneously hypertensive rats (SHR), respectively. In Figure 2A, the symbol (***) indicates that p <0.0001 vs. vehicle Ang-(1-7); the symbol (###) indicates that p <0.0001 vs. vehicle Arg0-Ang-(1-7); and Ns = no significance. In Figure 2B, the symbol (*) indicates that p <0.05 vs. vehicle Ang-(1-7); the symbol (***) indicates that p <0.0001 vs. vehicle Arg0-Ang-(1-7); and the symbol (###) indicates that p <0.0001 arg0-Ang-(1-7) vs. Ang (1-7). Figure 2C shows the concentration-dependent vasodilatation effect of arg0-Ang-(1-7) and Ang-(1-7) peptides in the mesenteric artery rings of SHR. Each point shows the mean ± standard error of the mean. The symbol (***) indicates that p <0.0001 Ang-(1-7) vs. Arg0-Ang (1-7).

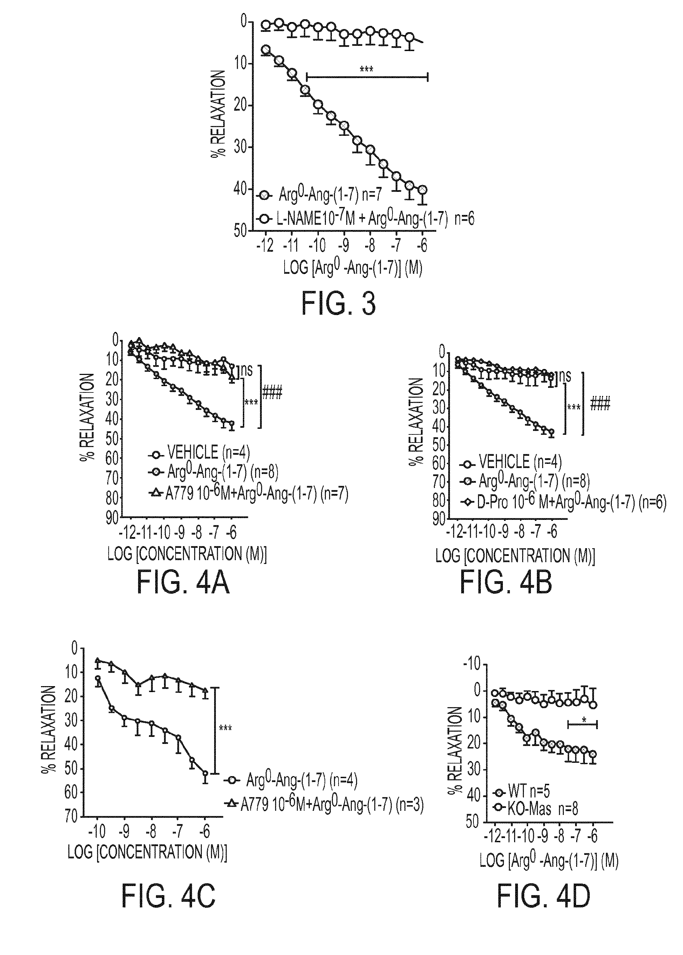

FIG. 3 shows the blockade of the vasodilatation effect of arg0-Ang-(1-7) after the inhibition of the production of nitric oxide by treatment with L-NAME in SHR rat aortic rings. Each point represents the mean ± standard error of the mean. Legend: the symbol (***) indicates that p <0.0001 for L-NAME + 10-7M arg0-Ang-(1-7) vs. Arg0-Ang-(1-7).

FIG. 4A and 4B show the effect of A-779 and D-Pro, respectively, in thoracic aortic rings of SHR rats immediately before adding the peptide arg0-Ang-(1-7) in increasing cumulative concentration. In Figure 4A, the symbol (***) indicates that p <0.0001 for A779 + 10-6M arg0-Ang-(1-7) vs. Arg0-Ang-(1-7); the symbol (###) indicates that p <0.0001 vs vehicle-arg0 Ang-(1-7); and Ns = no significance. In Figure 4B, the symbol (***) indicates that p <0.0001 for D-Pro-arg0 10-6M + Ang-(1-7) vs. Arg0-Ang-(1-7); the symbol (###) indicates that p <0.0001 vs. vehicle-arg0 Ang-(1-7); and ns = no significance. Figure 4C shows the effect of A-779 in the mesenteric artery rings SHR. Each point shows the mean ± standard error of the mean. The symbol (***) indicates that p <0.0001 for A779 10-6M + arg0-Ang-(1-7) vs. Arg0-Ang-(1-7). Figure 4D shows the blocking effect of the arg0-Ang-(1-7) vasodilation in a knockout mouse aortic rings to the Mas receptor of angiotensin-(1-7) (KO-Mas). Each point represents the mean ± standard error of the mean. The symbol (*) indicates that p <0.05 compared to animal control wild type (WT).

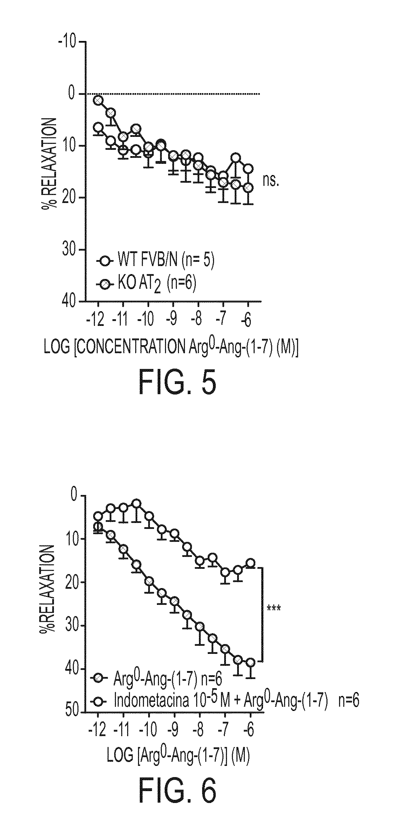

FIG. 5 shows the concentration-dependent vasodilatation effect induced by arg0-Ang-(1-7) in aortic rings of a knockout mouse for the AT2 receptor (AT2-KO). Each point represents the mean ± standard error of the mean. The abbreviation ns indicates no significant difference between the animals KO-AT2 and WT.

FIG. 6 shows the blocking of the vasodilatation effect of arg0-Ang-(1-7) after prostaglandin inhibition by indomethacin treatment in aorta rings of SHR rats. Each point represents the mean ± standard error of the mean. Legend: the symbol (***) indicates that p <0.0001 for Indomethacin 10-5 M + arg0-Ang-(1-7) vs. Arg0-Ang-(1-7).

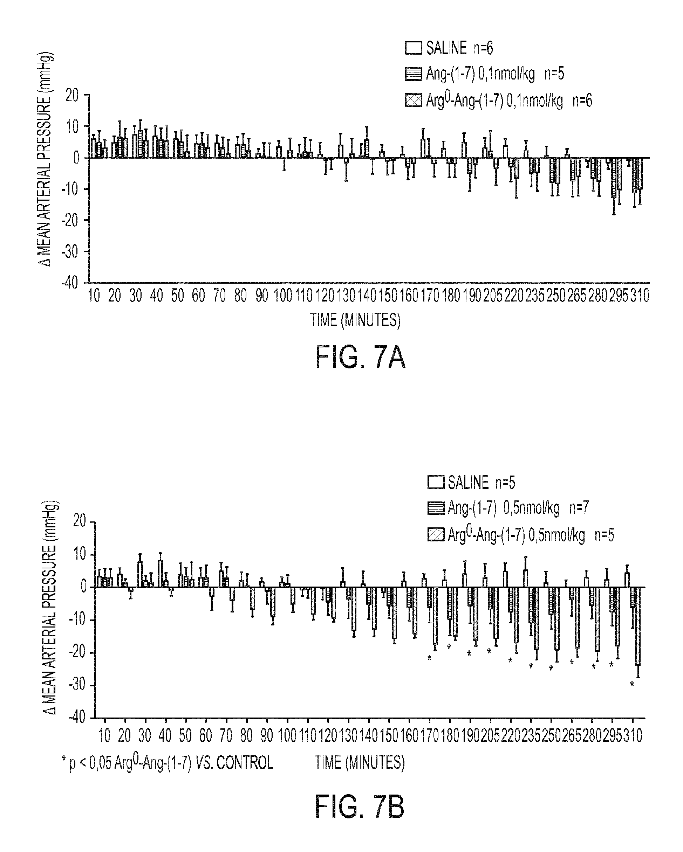

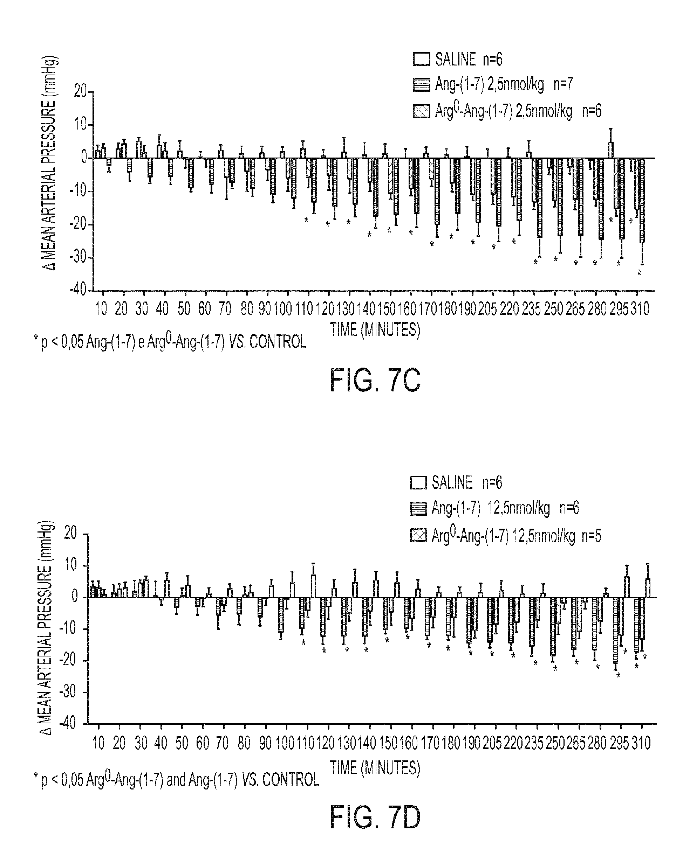

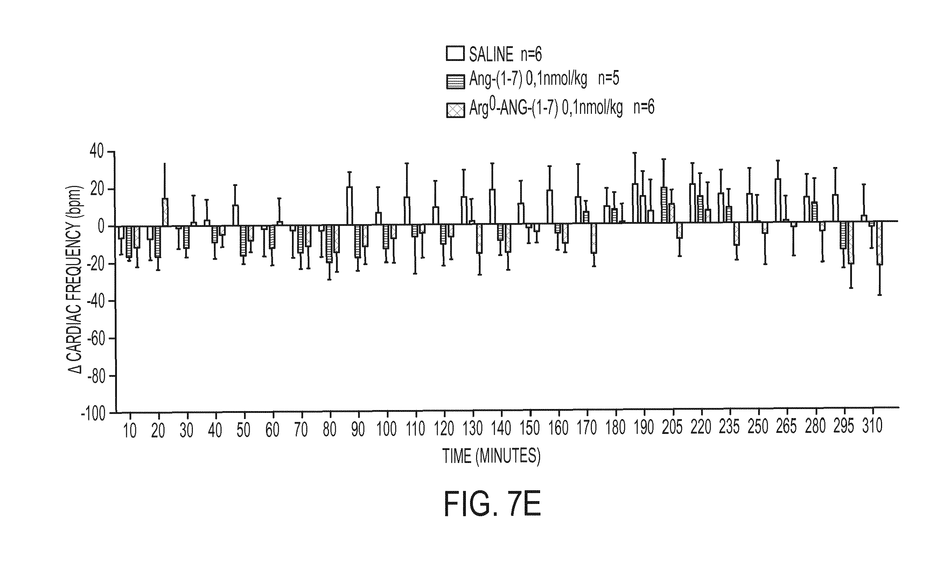

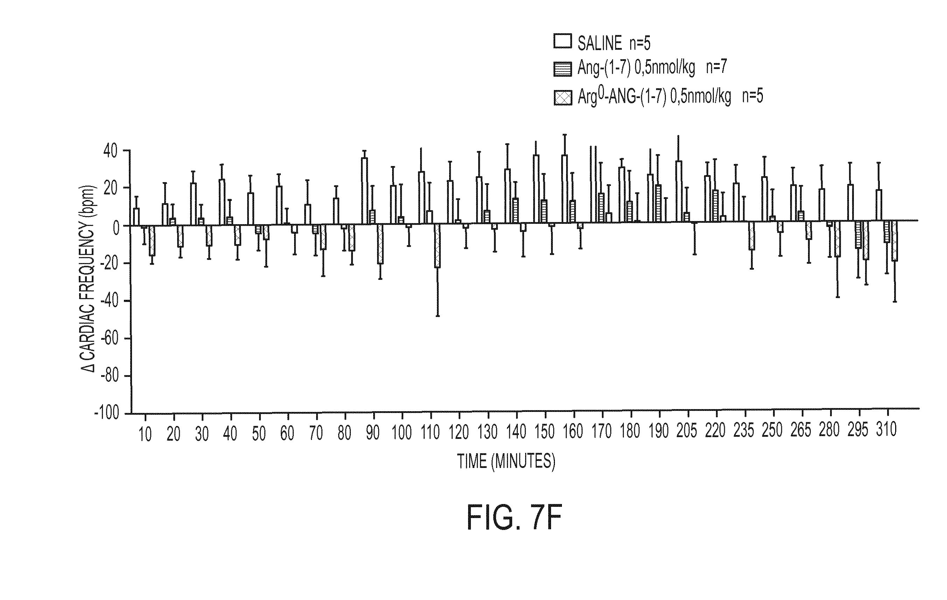

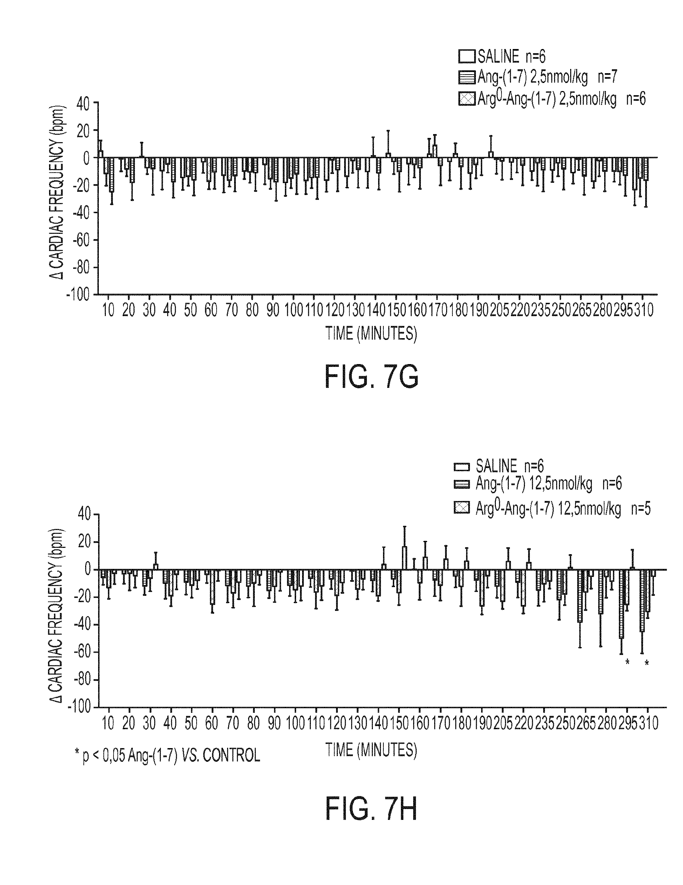

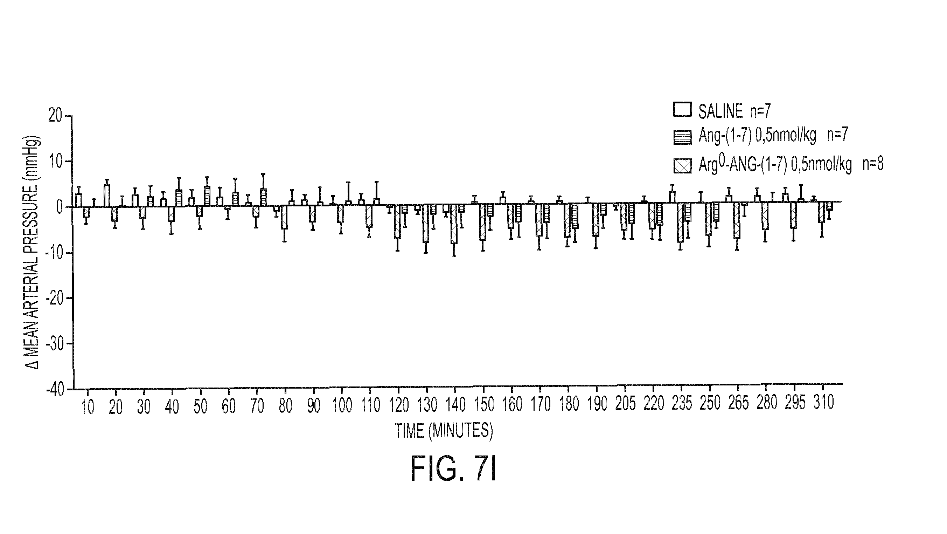

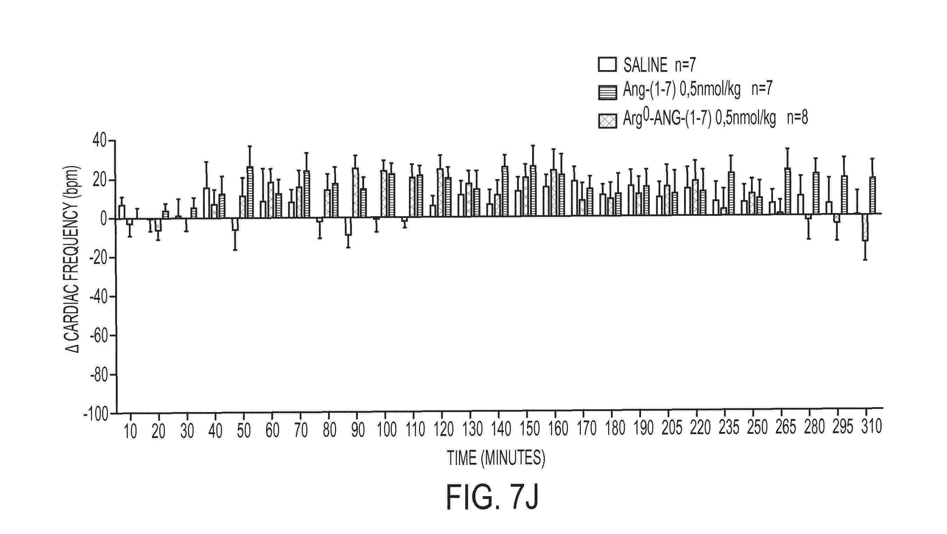

FIG. 7A-1 to 7B-4 show the reduction of time and dose-dependent mean arterial pressure (MAP) after acute administration in bolus of arg0-Ang-(1-7) and Ang-(1-7) peptides in doses of 0.1 nmol/kg (Figure 7A-1); 0.5 nmol/kg (Figure 7A-2); 2.5 nmol/kg (7A-3) and 12.5 nmol/kg (Figure 7A-4) in conscious SHR. Statistics: Figure 7A-2, the symbol (*) indicates p <0.05 arg0-Ang-(1-7) vs. saline. In Figure 7A-3, the symbol (*) indicates p <0.05 arg0-Ang-(1-7) and Ang-(1-7) vs. saline. In Figure 7A-4, the symbol (*) indicates p <0.05 for arg0-Ang-(1-7) and Ang-(1-7) vs. saline. In Figures 7B-1, 7B-2, 7B-7B-3 and 4, the data show no change in heart rate after administration, under the same conditions, of arg0-Ang-(1-7) and Ang-(1-7) peptides. Each point shows the mean ± standard error of the mean.

FIG. 7C-1 and 7C-2 show no reduction of mean arterial pressure and heart rate after administration of the bolus of arg0-peptide Ang-(1-7) and Ang-(1-7), at a dose 0.5 nmol/kg, on awake Wistar rats. Each point shows the mean ± standard error of the mean.

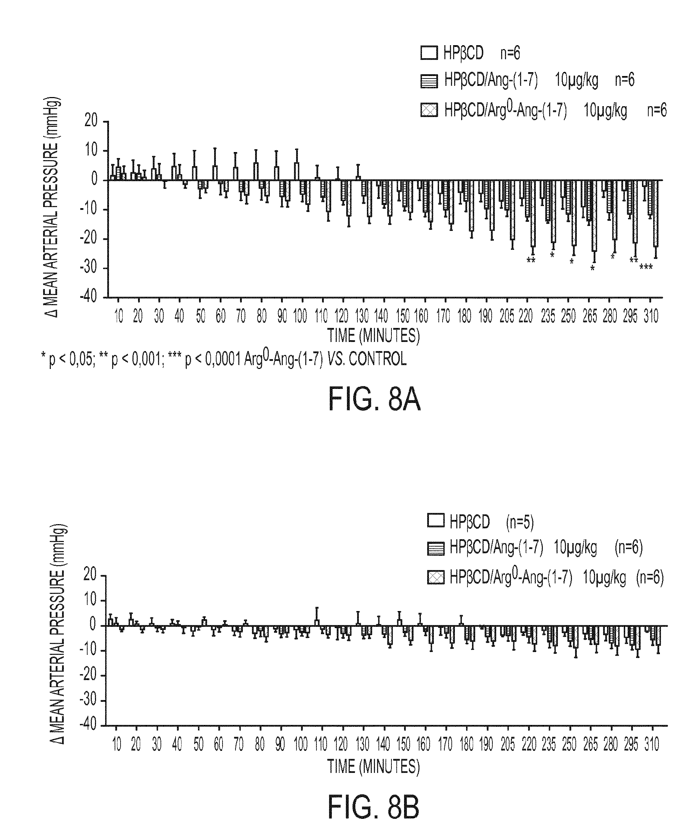

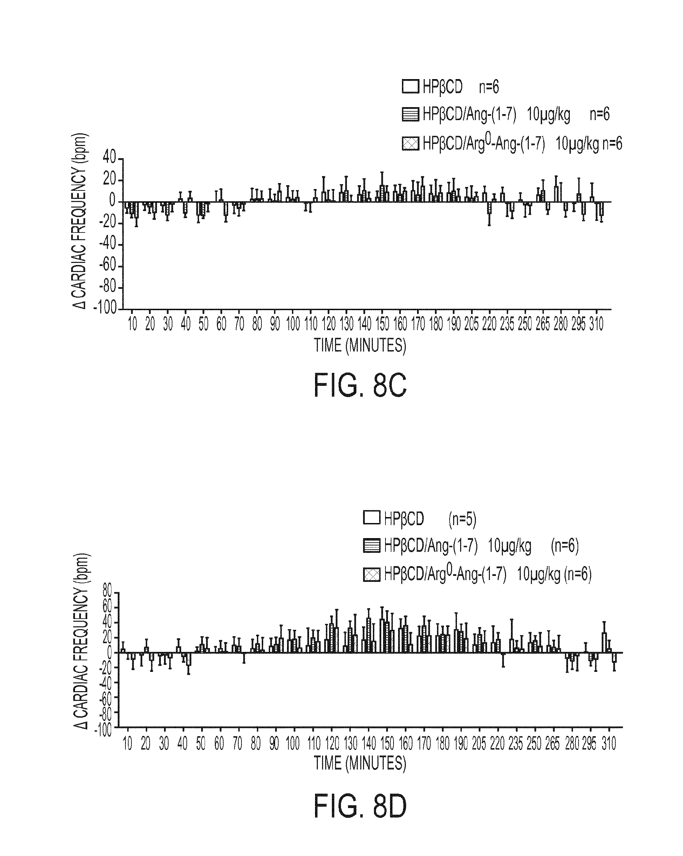

FIG. 8A-1 and 8B-1 show the time-dependent decrease in MBP after oral administration of a single dose (10ug/kg) of arg0-Ang-(1-7) and Ang-(1-7) in cyclodextrin in conscious SHR. Statistics: Figures 8A-1, the symbol (*) indicates p <0.05 for arg0-Ang-(1-7) vs. HPβCD (**) p <0.001 for arg0-Ang-(1-7) vs. HPβCD and (***) p <0.0001 arg0-Ang-(1-7) vs. HPβCD. On the other hand, Figures 8B-1 shows no change in heart rate after administration, under the same conditions, for arg0-Ang-(1-7) and Ang-(1-7) peptides. Each point shows the mean ± standard error of the mean. Figures 8A-2 and 8B-2 show no decrease in MAP and heart rate following oral administration of a single dose (10ug/kg) of arg0-Ang-(1-7) or Ang-(1 -7) included in cyclodextrin in awake Wistar rats. Each point shows the mean ± standard error

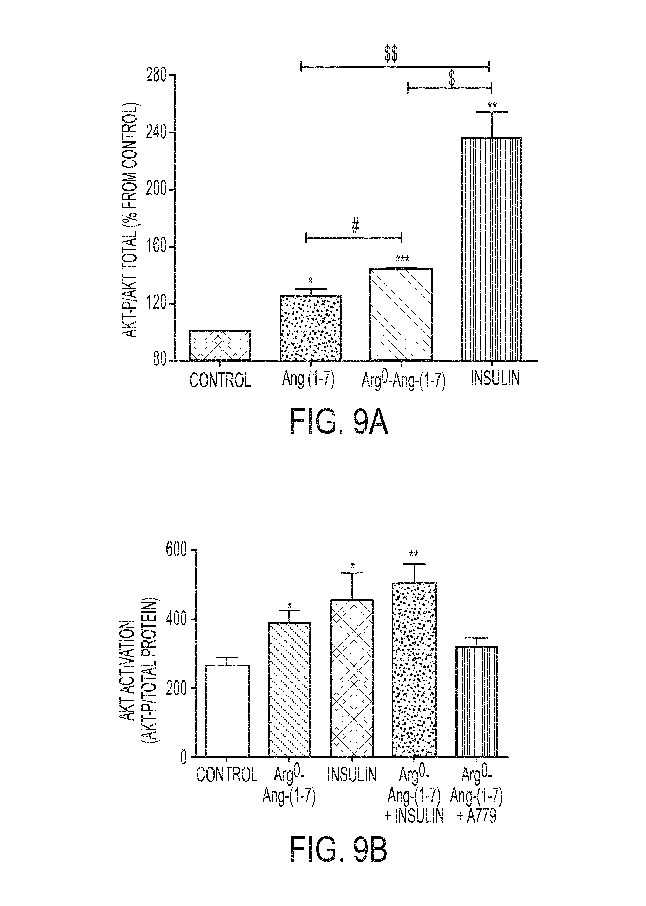

FIG. 9A shows the results obtained for the activation (phosphorylation) of AKT protein in the presence of peptide arg0-Ang-(1-7), Ang-(1-7) and insulin (positive control) in cardiomyocytes in cell culture. Statistical data: *p <0.05 vs. control Ang-(1-7); **p<0.01 vs. control Insulin; ***p<0.0001 vs. control Arg0-Ang-(1-7); #p <0.05 Ang-(1-7) vs. Arg0-Ang-(1-7); $p<0.05 insulin vs. Arg0-Ang-(1-7); $$p<0.01 insulin vs. Ang-(1-7). Figure 9B shows the results obtained for the effect of the Ang-arg0-(1-7) peptide, the activation of AKT in primary cell culture pre-adipocytes extracted from epididymal adipose tissue from adult male Wistar rats. The data represent the mean + standard error of the mean analyzed by Student's t test (*p<0.05 and **p<0.01 vs. control), n = 6.

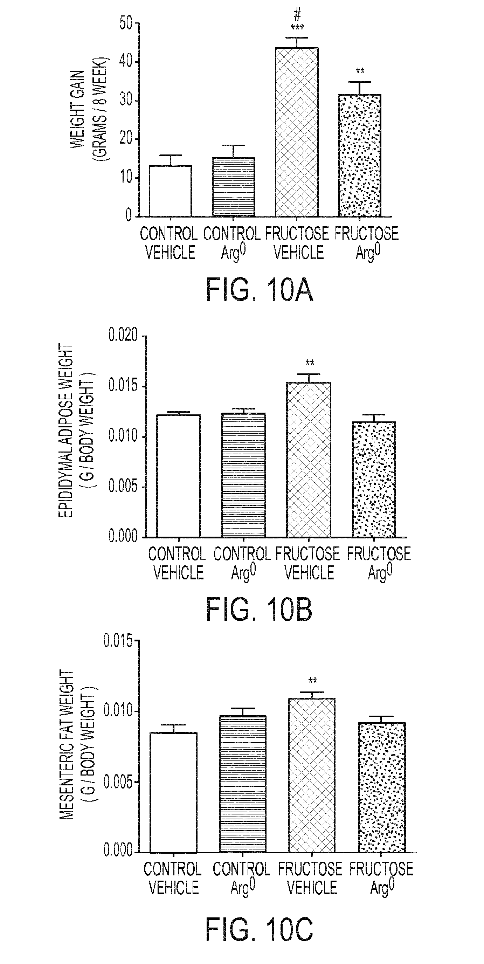

FIG. 10 shows the results obtained for the evaluation of the body weight gain, visceral adiposity in metabolic syndrome model treated with arg0-angiotensin-(1-7). Male Sprague-Dawley rats fed (fructose) or not (control) with 10% fructose in water and treated (arg0) or not (Vehicle) with arg0-Ang-(1-7) with cyclodextrin were subjected to the evaluation of the body weight gain (A) and mass increase of epididymal adipose tissue (B) and mesenteric adipose tissue(C). The data represent the mean + standard error of the mean analyzed by two-way ANOVA (*p<0.05, **p<0.01 and ***p<0.001 versus controls (A) and versus the other groups (B and C); #p<0.05 versus treatment), n = 6.

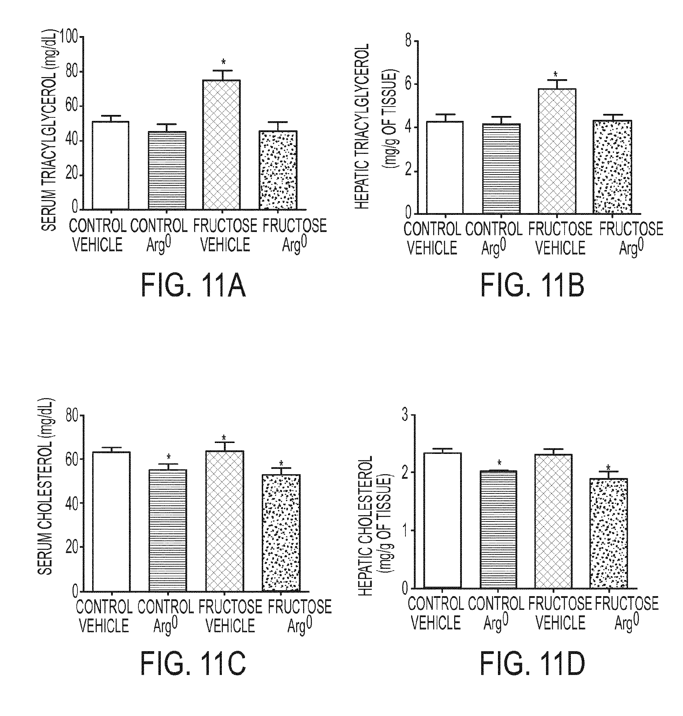

FIG. 11 shows the results obtained for the determination of serum and hepatic levels of triacylglycerol and cholesterol in a metabolic syndrome model treated with arg0-angiotensin-(1-7).Male Sprague-Dawley rats, fed with 10% fructose in water (fructose) or not (control) and treated with arg0-Ang-(1-7) with cyclodextrin (arg0) or not (vehicle), were assessed regarding the levels of serum triglyceride (A), liver triglyceride (B), serum cholesterol (C) and liver cholesterol (D) after 6 hours of fasting. The data represent the mean + standard error of the mean analyzed by two-way ANOVA (A and B) and Student's t test (C and D) (*p<0.05 versus all other groups), n = 6.

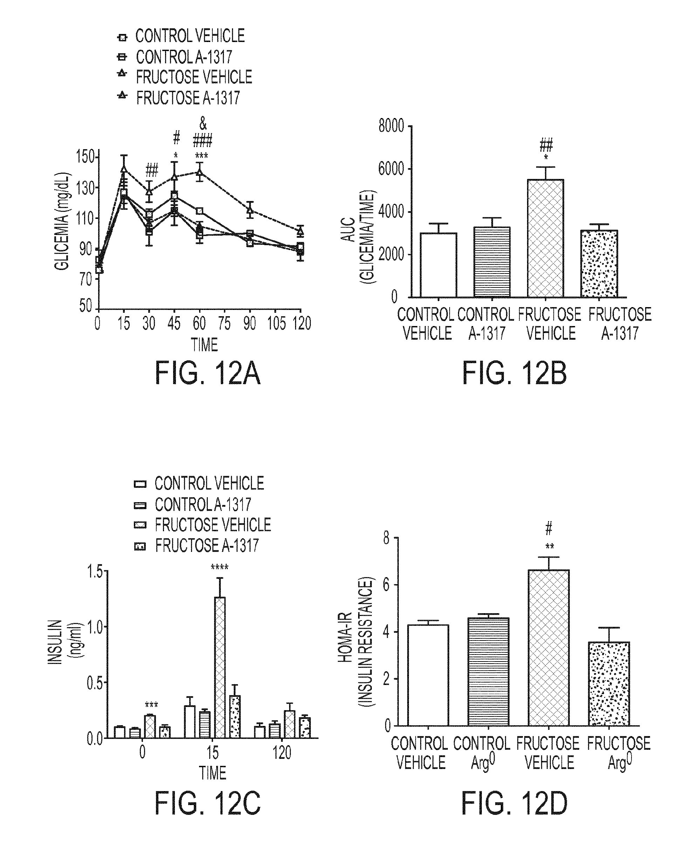

FIG. 12 shows the results obtained for assessment of glucose tolerance and insulin resistance in a metabolic syndrome model treated with arg0-angiotensin-(1-7). Male Sprague-Dawley rats fed (fructose) or not (control) with 10% fructose in water and treated (arg0) or not (Vehicle) with arg0-Ang-(1-7) with cyclodextrin were subjected to an oral glucose tolerance test after 12 hours of fasting. Blood glucose was measured at 0 (baseline), 15, 30, 45, 60, 90 and 120 minutes after oral administration of glucose (2g/kg body weight), insulin release was assessed at 0 (baseline), 15 and 120 minutes of the experimental protocol, and the HOMA-IR index was assessed by blood glucose and fasting insulin (6-8 hours). Blood glucose data by the time (A), area under the curve (B), release of insulin (C) and HOMA-IR index (D) represent the mean + standard error of the mean, analyzed by two-way ANOVA (*p< 0.05, **p<.01, ***p<0.001, and ****p<0.0001 vs. versus other treatment groups, or (C); #p<0.05, ##p<0 01 and ###p<0.001 vs. control vs. controls or arg0 (A); &p<0.05 vs. vehicle control (A)), n = 6.

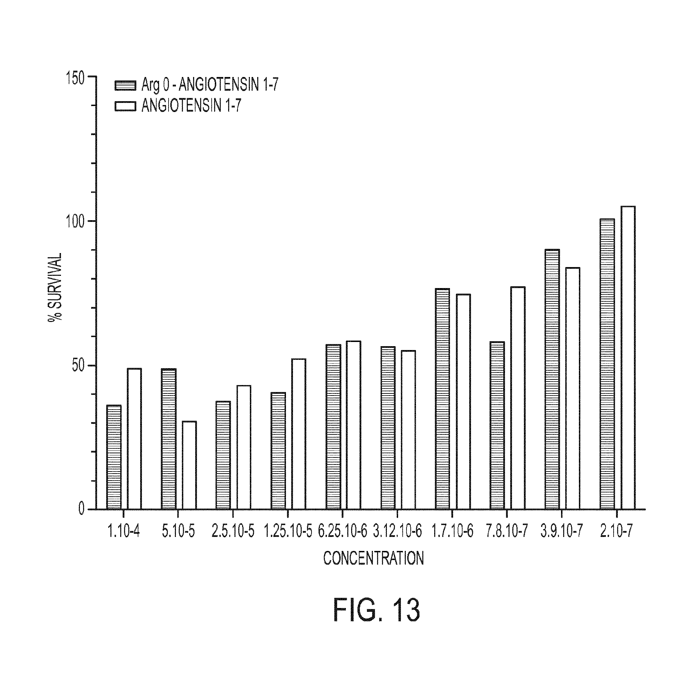

FIG. 13 shows the anti-proliferative effect of arg0-Ang-(1-7) and Ang-(1-7) peptides in human alveolar adenocarcinoma cells (A-549).

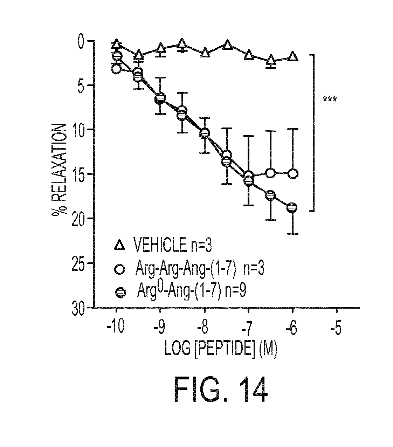

FIG. 14 shows the concentration-dependent vasodilatation effect of arg0-Ang-(1-7) and Arg-Arg-Ang-(1-7) peptides in aortic rings of Wistar rat. The symbol (**) indicates p <0.001 arg0-Ang-(1-7) compared to the control. Each point shows the mean ± standard error of the mean.

### DETAILED DESCRIPTION OF THE INVENTION

The present invention consists of the peptide (arg0) n-Ang-(1-7), where n is 1 to 10 (SEQ ID N):1), where Xaa represents 1 to 10 L-Arg, which is produced by adding at least one amino acid arginine, up to 10 amino acid arginine, at the amino terminal position of Ang-(1-7). This peptide can be used for treating or preventing diseases or cardiopulmonary and liver disorders, vascular disorders, metabolic disorders, neural disorders, genito-urinary tract disorders, musculoskeletal disorders, skeletal disorders, renal disorders, skin disorders, alopecia or tumors. The peptide (arg0) n-Ang-(1-7) is able to activate the protein kinase B (AKT), which is key to the metabolic actions of insulin and to increase the production of nitric oxide (NO) via AKT-dependent means. Consequently, the peptide causes, for example, dilation of aortic and mesenteric vessels, reduction of mean arterial pressure and an anti-proliferative effect.

The invention also encompasses pharmaceutical formulations comprising this peptide. The peptide may be prepared in solid form (including pellets, powders or suppositories) or liquid form (e.g., solutions, suspensions and emulsions) and can be subjected to operations such as sterilization and may contain pharmaceutical and pharmacologically acceptable excipients, such as stabilizers, wetting agents, emulsifiers, preservatives, co-solvents, suspending agents, thickening agents, components for adjusting osmolarity and ionic strength and other excipients, in addition to buffering systems. Other suitable excipients are lactose, sucrose, starch powder, cellulose esters of alkanoic acids, stearic acid, talc, magnesium stearate, magnesium oxide, sodium and calcium salts of phosphoric and sulfuric acids, acacia, gelatin, alginate sodium, polyvinylpyrrolidone and/or polyvinyl alcohol, and prepared as tablet or encapsulated for conventional administration.

Water soluble preservatives which may be used in the vehicle include sodium bisulfite, sodium thiosulfate, ascorbate, benzalkonium chloride, chlorobutanol, thimerosal, phenylmercuric borate, parabens, benzyl alcohol, phenylethanol or antioxidants such as Vitamin E, tocopherol, and chelating agents such as EDTA and EGTA.

Alternatively, the peptide of this invention can be dissolved in saline, water, polyethylene glycol, polypropylene glycol, carboxymethyl cellulose colloidal solutions, hydroxyethyl cellulose, carbopol®, ethanol, ethyl oleate, triglycerides, vegetable oil, tragacanth gum, and/or various buffers. The carrier or diluent may include sustained release materials, such as glyceryl monostearate, glyceryl distearate alone or with wax, or other materials known in the art.

The peptide can be administered by oral, local, transdermal, topical, intranasal, ocular, parenteral, subcutaneous, intravenous, intramuscular, intraperitoneal, intrasternal, intratendinous, intraspinal, intracranial, and intrathoracic routes.

Conventional forms for topical administration include wound dressings, bandages, polymeric films, slurries, ointments, lotions, creams, gels, sprays, transdermal patches and aerosols. The transdermal passage of peptide may be increased through the use of permeation enhancers or nanocarriers such as liposomes, niosomes and cyclodextrins and ultradeformable lipid vesicles. The skin permeation enhancers classically used in the art include, but are not limited to, terpenes, terpenoids, essential oils, pyrrolidones and derivatives, fatty acids and esters, sulfoxides, and the like, alcohols, glycols, glycerides, N-dodecyl, N-dimethylamino acetate.

Solid dosage forms for oral administration include capsules, tablets, gelatin capsules, granules and powders. In these formulations, the peptide is admixed with at least one diluent such as sucrose, lactose or starch. This formulation may further contain additional substances, e.g., lubricating agents such as magnesium stearate, disintegrants, binders or surfactants, chitosan, cyclodextrin or its derivatives as penetration enhancer. In the case of capsules, tablets or gelatin capsules, the formulation may also contain buffering systems. The tablets and capsules may further be prepared with an enteric coating.

Liquid forms for oral administration may include pharmaceutically acceptable emulsions, solutions, suspensions, syrups and elixirs. These compositions may also include excipients such as wetting agents, emulsifiers, sweeteners and perfumes.

The peptide may also be associated with carrier systems and/or controlled drug release systems in order to increase peptide stability, higher bioavailability, a more prolonged action and/or targeting to the target tissue. These carrier systems or controlled release classically used in the art include, but are not limited to, cyclodextrins, polymers, mucoadhesive polymers, lipid vesicles, liposomes, polymersomes, solid lipid nanoparticles, polymeric micro- and nanoparticles, micro-and nanocapsules, micro- and nanoemulsions, dendrimers, micelles, polymeric micelles, inorganic nanoparticles, carbon nanoparticles, transdermal patches, implantable polymeric matrices and devices that may be deployed.

Furthermore, the peptide object of the invention can be used in combination with or in place of other drugs.

The invention may be better understood, in a non-limiting way from the following examples.

### Example 1 - Effect of arg0-Ang-(1-7) on Nitric Oxide Production.

Chinese hamster ovary cells (CHO) were stably transfected with the cDNA of the rat receptor Mas driven by a cytomegalovirus promoter and selected using neomycin. Nitric oxide production was monitored using the indicator NO 4-amino-5-methylamino-2', 7'-difluorofluorescent diacetate.

Confluent CHO cells transfected with the Mas receptor and cultured in 6-well plates were preincubated in 1.5 ml of serum free medium (DMEM) containing DAF-FM (least 5 uM) and supplemented with 0.2% BSA, 0.005% bacitracin, 0.1 mol l-1 phenylmethylsulfonyl fluoride and 0.5 mol/L 1, 10-phenanthroline at 37 ° C in a humidified incubator in an atmosphere with 5% CO₂. After 30 minutes, cells were washed and incubated with arg0-Ang-(1-7) and Ang-(1-7) at 10⁻⁷ mol/L for 60 minutes. Subsequently, the cells were washed twice and the slides were mounted for confocal microscopy for evaluation.

Relative fluorescence measurements were made using a Zeiss LSM 510 laser scanning META confocal microscope excited at 488 nm with an argon-ion laser (objective lens immersion oil, 63X).

Results are presented as mean ± SEM. One-way analysis of variance with Bonferroni post-test for multiple comparisons was used to compare the concentration-response curve in CHO cells transfected with the receptor Mas. All statistical analyses were considered significant when p <0.05.

Arg0-Ang-(1-7) and Ang-(1-7) stimulated nitric oxide production in Chinese hamster ovary cells transfected with the Mas receptor. Notably, arg0-Ang-(1-7) more markedly stimulated nitric oxide production in CHO cells transfected with the receptor Mas at a concentration of 10⁻⁷ mol/L when compared with Ang-(1-7) (Figure 1).

### Example 2 - Vasorelaxant Effects of arg0-Ang-(1-7) on Aortic and Mesenteric Vessels.

Three- to four-mm rings of the descending thoracic aorta, free from adipose and connective tissue, were maintained in a gasified solution (95% 02 and 5% CO2) of Krebs-Henseleit, at 37 ° C under 1.0 g tension, 1 hour for stabilization. The presence of functional endothelium was assessed by the ability of acetylcholine [ACh] (10 µmol/L) to induce more than 70% relaxation of vessels pre-contracted with phenylephrine (Phe) (0.1 µmol/L). The mechanical activity, recorded isometrically by a force transducer, was fed into a recorder-amplifier and a computer equipped with an analog-digital converter board, using CVMS data acquisition/recording software.

The vessels from normotensive Wistar rats and spontaneously hypertensive rats (SHR) were pre-contracted in the same voltage level (approximately 1.0 g tension) at the submaximal concentration of phenylephrine (0.1 µmol/L). Arg0-Ang-(1-7) and Ang-(1-7) were added in increasing cumulative concentrations as the response to phenylephrine was stabilized. Results are presented as mean ± SEM. One-way analysis of variance (ANOVA) with Newman-Keuls post test of multiple comparisons was used to compare the concentration response curves obtained in the aortic rings. The vasodilatation effect of arg0-Ang-(1-7) and Ang-(1-7) was expressed as the percentage of decrease in peak concentration induced by phenylephrine. All statistical analyses were considered significant when p <0.05.

Arg0-Ang-(1-7) and Ang-(1-7) produced vasorelaxation in aortic rings from normotensive Wistar and SHR rats, and the Emax of arg0-Ang-(1-7) (Emax = 18.97% ± 1:33 Wistar rats rings and 38.0 ± 3.3% in SHR rings) was greater than that of Ang-(1-7) (Emax = 12:46 ± 0.98% in rats rings and 30 ± 3% in SHR rings ).

The results, shown in Figures 2A and 2B show the vasorelaxant effect produced by arg0-Ang-(1-7) is endothelium-dependent, similar to the vasorelaxant effect produced by Ang-(1-7).

Rings (2.0 mm) of mesenteric artery (2nd and/or 3rd branches) in spontaneously hypertensive rats (SHR), free from adipose and connective tissue, were maintained in a gasified solution (95% 02 and 5% CO2) of Krebs-Hanseleit to 37°C. After a 15 minute stabilization period, the artery was stretched, gradually, to a resting tension considered optimal with respect to its inner diameter. In each arterial segment, the voltage/diameter was calculated by the internal circumference which corresponds to a transmural pressure of 100 mmHg for a relaxed vessel in situ (IC100) using software specific for standardization resistance arteries. For the experiments, the arteries were maintained with an internal circumference, IC1, calculated using the formula IC1 = 0.90 x IC100, in which the development of strength is maximum, as previously described by Mulvany and Halpern (1977). The effective luminal diameter was determined according to the I1 = IC1/π equation. The diameter of the vessels used ranged from 200 to 300µm.

The arteries were stimulated with a high concentration of potassium chloride (KCl 120 mmol/l), in order to assess their functional integrity. The administration of KCl was performed twice, with 15-minute break to reproduce the contractile response. Preparations reaching a contraction equal to or greater than 6 mN/mm were considered viable. Preparations showing less contraction than 6 mN/mm were discarded.

The presence of functional endothelium was assessed by the ability of acetylcholine [ACh] (0.1 µmol/L) to induce more than 60% of relaxation in vessels precontracted with phenylephrine (0.1 µmol/L). The mechanical activity was recorded isometrically using a myograph, and this was connected to a system for data acquisition and a computer.

The SHR vessels were pre-contracted with sub-maximal concentrations of phenylephrine (0.1 µmol/L) and arg0-Ang-(1-7) and Ang-(1-7) peptides were added in increasing cumulative concentrations as the response to phenylephrine was stabilized. Results are presented as mean ± SEM. Two-away of variance (ANOVA) with Bonferroni's multiple comparison post-test was used to compare the concentration response curves obtained in mesenteric rings. The vasodilatation effects of arg0-Ang-(1-7) and Ang-(1-7) were expressed as the percentage of decrease in peak concentration induced by phenylephrine. All statistical analyses were considered significant when p <0.05.

Arg0-Ang-(1-7) and Ang-(1-7) produced vasorelaxation in SHR mesenteric rings, and the Emax of arg0-Ang-(1-7) (Emax = 51.7 rings in rats SHR) was higher than that of Ang-(1-7) (Emax = 28.0 rings in SHR rats) (Figure 2C).

### Example 3 - Involvement of Nitric Oxide in the Arg0 Vasodilatation Effect of Ang-(1-7) in SHR Rat Aortic Rings.

To assess whether the nitric oxide synthase (NOS) participates in the vasodilatation effect induced by arg0-Ang-(1-7), vessels containing functional endothelium were pre-incubated with N^{G}-Nitro-L-Arginine Methyl Ester (L- NAME), an inhibitor of NOS, to 10⁻⁷ M for 20 minutes. Then the vessels were pre-contracted with Phe 10⁻⁷ M after stabilization of contraction, and cumulative concentration-response curves (10⁻¹² to 10⁻⁶ M) were constructed with the arg0-Ang-(1- 7). As a positive control, other vascular segment was simultaneously monitored in the presence of only arg0-Ang-(1-7).

Results are presented as mean ± standard error of the mean. Two-way analysis of variance (ANOVA) with Bonferroni's multiple comparison post-test was used to compare the concentration response curves obtained in the aortic rings. The vasodilatation effect of arg0-Ang-(1-7) was expressed as the percentage of decrease in peak concentration induced by phenylephrine. All statistical analyses were considered significant when p <0.05.

The result, shown in Figure 3, indicates that the vasorelaxant effect produced by arg0-Ang-(1-7) was completely blocked in the presence of L-NAME at 10⁷ M, and thus were dependent on nitric oxide production.

Example 4 - Mas receptor Performance on the Vasorelaxant Effect of arg0-Ang-(1-7) in Aortic and Mesenteric Vessels.

Three- to four-mm rings of the descending thoracic aorta in spontaneously hypertensive rats (SHR), free from adipose and connective tissue, were maintained in a gasified solution (95% 02 and 5% CO2) of Krebs-Henseleit, at 37 °C under a tension of 1.0 g for 1 hour for stabilization. The presence of functional endothelium was assessed by the ability of acetylcholine [ACh] (10 µmol/L) to induce more than 70% relaxation of vessels pre-contracted with phenylephrine (0.1 µmol/L). The mechanical activity, recorded isometrically by a force transducer, was fed into a recorder-amplifier and a computer equipped with an analog-digital converter board, using CVMS data acquisition/recording software.

The SHR vessels were pre-contracted to the same voltage level (approximately 1.0 g tension) at the submaximal concentration of phenylephrine (0.1 µmol/L). Mas receptor antagonists, A-779 and D-Pro, were added to 10-6 µmol/L immediately before adding the arg0-Ang-(1-7) in increasing cumulative concentrations. Results are presented as mean ± SEM. One-way analysis of variance (ANOVA) with the Newman-Keuls post-test for multiple comparisons was used to compare the concentration response curves obtained in the aortic rings. The result was expressed as the percentage of decrease in peak concentration induced by phenylephrine. All statistical analyses were considered significant when p <0.05.

The vasorelaxation produced by arg0-Ang-(1-7) was blocked in SHR rat aortic rings in the presence of A-779 and D-Pro (779 A-E max = 14.0 ± 1.2%; Emax = D-Pro 11 ± 1.5%) (Figures 4A and 4B). These results show that Ang-arg0-(1-7) peptide acts via the Mas receptor since the specific Mas receptor antagonists, A-779 and D-Pro, blocked the vasorelaxant effect of the peptide.

Rings (2.0 mm) of mesenteric artery (2nd and/or 3rd branches) in spontaneously hypertensive rats (SHR), free from adipose and connective tissue, were maintained in a gasified solution (95% 02 and 5% CO2) of Krebs-Hanseleit, at 37 °C. After a 15 minute stabilization period, the artery was stretched, gradually, to a resting tension considered optimal with respect to its inner diameter. For this, in each arterial segment, the voltage/diameter was calculated by internal circumference which corresponds to a transmural pressure of 100 mmHg for a relaxed vessel in situ (IC100) using software specific for standardization resistance arteries. For the experiments, the arteries were maintained with an internal circumference, IC1, calculated using the formula IC1 = 0.90 x IC100, in which the development of strength is maximum, as previously described by Mulvany and Halpern (1977). The effective luminal diameter was determined according to the 11 = IC1/π equation. The diameter of the vessels used ranged from 200 to 300 µm.

The arteries were stimulated with high concentration of potassium chloride (KCl 120 mmol/L), in order to assess their functional integrity. The administration of KCl was performed twice, with a 15-minute break to check the contractile response. Preparations reaching a contraction equal to or greater than 6 mN/mm were considered viable. Preparations showing less contraction than 6 mN/mm were discarded.

The presence of functional endothelium was assessed by the ability of acetylcholine [ACh] (0.1 µmol/L) to induce more than 60% relaxation in vessels precontracted with phenylephrine (0.1 µmol/L). The mechanical activity was recorded isometrically using a myograph, which was connected to a system for data acquisition and to a computer.

The SHR vessels were pre-contracted with sub-maximal concentrations of phenylephrine (0.1 µmol/L). Mas receptor antagonist, A-779, was added to 10⁻⁶ µmol/L immediately before adding the arg0-Ang-(1-7) in increasing cumulative concentrations. Results are presented as mean ± SEM. Two-way analysis of variance (ANOVA) with Bonferroni's multiple comparison post-test was used to compare the concentration response curves obtained in mesenteric rings. The result was expressed as a percentage of decrease in peak concentration induced by phenylephrine. All statistical analyses were considered significant when p <0.05.

The vasorelaxation produced by arg0-Ang-(1-7) was blocked in SHR mesenteric rings in the presence of A-779 (779 A-E max = 14.1) (Figure 4C). These results show that Ang-arg0-(1-7) peptide acts via the Mas receptor since the specific Mas receptor antagonist A-779 blocked the vasorelaxant effect of the peptide.

### a) Effect of arg0-Ang-(1-7) in mouse aortic rings with genetic deletion of the Mas receptor (KO-Mas)

To confirm the involvement of the Mas receptor, the vasodilatation effect induced by arg0-Ang-(1-7) was used for rat aortic rings Mas-KO and WT as a control. The vessels with functional endothelium were pre-contracted with Phe, and during the tonic phase of the contraction a cumulative concentration-response curve was constructed (10⁻¹² to 10⁻⁶ M) with arg0-Ang-(1-7).

Results are presented as mean ± standard error of the mean. Two-way analysis of variance (ANOVA) with Bonferroni's multiple comparison post-test was used to compare the concentration response curves obtained in the aortic rings. The vasodilatation effect of arg0-Ang-(1-7) was expressed as the percentage of decrease in peak concentration induced by phenylephrine. All statistical analyses were considered significant when p <0.05.

As can be seen in Figure 4D, there was no vasodilatation effect of peptide arg0-Ang-(1-7) in aorta rings of mice Mas-KO. This result confirms data obtained with pharmacological blockade Mas receptor antagonists with A-779 and D-Pro. Thus, it is concluded that arg0-Ang-(1-7) acts via the Mas receptor, since in mice with Mas receptor deletion the vasorelaxant effect of the peptide was blocked.

### Example 5 - Effect of arg0-Ang-(1-7) in AortaRrings of Mice with Genetic Deletion of the AT2 Receptor (AT2-KO).

Mouse aortic rings of AT2 KO-containing functional endothelium were pre-contracted with Phe at 10⁻⁶ M. After stabilization of the contraction, a cumulative concentration-response curve (10⁻¹² to 10⁻⁶ M) was constructed with arg0-Ang-(1-7).

Results are presented as mean ± standard error of the mean. Two-way analysis of variance (ANOVA) with Bonferroni's multiple comparison post-test was used to compare the concentration response curves obtained in the aortic rings. The vasodilator effect of arg0-Ang-(1-7) was expressed as the percentage of decrease in peak concentration induced by phenylephrine. All statistical analyses were considered significant when p <0.05.

As can be seen in Figure 5, the vasorelaxant effect induced by arg0-Ang-(1-7) was not inhibited in the absence of the AT2 receptor. The result shows that peptide arg0-Ang-(1-7) does not act via AT2 receptor.

### Example 6 - Involvement of Prostaglandins in the Vasodilator Effect of arg0-Ang-(1-7) in SHR Rat Aortic Rings.

To assess whether the cyclooxygenase pathway participates in the vasodilator effect induced by arg0-Ang-(1-7), vessels containing functional endothelium were pre-incubated with indomethacin to 10⁻⁵ M for 20 minutes. Then the vessels were pre-contracted with Phe at 10⁻⁷ M. After stabilization of contraction, cumulative concentration-response curves (10⁻¹² to 10⁻⁶ M) were constructed with the arg0-Ang-(1-7). As a positive control, another vascular segment was simultaneously monitored in the presence of arg0-Ang-(1-7) only.

Results are presented as mean ± standard error of the mean. Two-way analysis of variance (ANOVA) with Bonferroni's multiple comparison post-test was used to compare the concentration response curves obtained in the aortic rings. The vasodilator effect of arg0-Ang-(1-7) was expressed as the percentage of decrease in peak concentration induced by phenylephrine. All statistical analyses were considered significant when p <0.05.

The result, shown in Figure 6, shows that after inhibition of prostaglandin production, the vasodilator effect of arg0-Ang-(1-7) was partially blocked in SHR rat aorta rings with functional endothelium and contracted with Phe. This result suggests that the vasodilation induced by arg0-Ang-(1-7) is partly dependent on the production of prostaglandins.

### Example 7 - Antihypertensive Effects of arg0-Ang-(1-7)

### a) acute IV administration in bolus

Wistar and SHR rats were anesthetized with 2.5% tribromoethanol, intraperitoneally (1 mL/100g). For femoral artery cannulation, cannulas made by connecting 15 cm polyethylene PE50 to 4 cm of PE10 polyethylene were used, filled with saline solution (NaCl 0.9%) and heparin (100 IU/0.1mL). After the animal was placed supine on the operating table, a trichotomy of the right inguinal region of the animal was performed. A small skin incision was made; the femoral neurovascular bundle was located and the femoral artery isolated. The cannula was introduced into the abdominal aorta through the femoral artery and was used for obtaining the cardiovascular parameters. A second cannula made by connecting 15 cm polyethylene PE50 to 2 cm of PE10 polyethylene, filled with saline (0.9% NaCl), was inserted into the femoral vein for administration of peptides. After cannulation, the catheters were secured and exteriorized subcutaneously into the back of the animal, in the cervical region. The incisions were sutured and the animals placed in individual cages, with free access to water and feed.

The values of mean arterial pressure (MAP) and heart rate (HR) were calculated from PA pulses and recorded by the data acquisition system (BIOPAC System, Model MP150). The pulsatile arterial pressure (PAP) was recorded by a signal sent to a transducer connected to the cannula inserted into the abdominal aorta via the femoral artery. The pressure oscillations were captured, amplified and converted by an analog/digital conversion board feeding signals to the data acquisition board. Through the card reader software, AcqKnowledge v.3.5.7 (Biopac System), PAP was continuously recorded with a sampling frequency of 2000-4000 Hz.

Twenty-four hours after implantation of the cannulas was conducted, the first protocol, which consisted of acute intravenous administration of peptides was performed. For this, during the experiment, the arterial catheter was connected to the pressure transducer for the beginning of the record. After 90 minutes of cardiovascular parameters stabilization (baseline), the administration of the peptides arg0-Ang-(1-7), Ang (1-7) or saline in a final volume of 300 uL was given in bolus. A dose-response curve (0.1 nmol/kg, 0.5 nmol/kg, 2.5 nmol/kg and 12.5 nmol/kg) was constructed for the purpose of obtaining the most effective dose arg0-Ang-(1-7). The Ang-(1-7) also was used to compare the effect induced by each peptide. Doses of 0.1 nmol/kg; 0.5 nmol/kg; 2.5 nmol/kg and 12.5 nmol/kg were administered to SHR (n = 5-7 for each dose) while awake. Only the most effective dose obtained with arg0-Ang-(1-7) was administered in Wistar rats (n = 7-8) while awake, with SHR as control.

After injection of the peptide or saline, cardiovascular parameters were continuously monitored for a period of 5 hours. The values of the MAP and HR were obtained every 10 minutes throughout the experimental period.

Results are presented as mean ± standard error of the mean. Two-way analysis of variance (ANOVA) with Bonferroni's multiple comparison post-test were used to compare the time-response curve obtained during the bolus administration in arg0-Ang-(1-7) and Ang-(1-7). All statistical analyses were considered significant when p <0.05.

The changes in MAP and HR after the acute intravenous administration of different doses of arg0-Ang-(1-7) and Ang-(1-7) in awake SHR rats, are shown in Figures 7A.1 the 7B.4. As can be seen, the intravenous administration of arg0-Ang-(1-7) and Ang-(1-7) induced a significant reduction in MAP which was time- and dose-dependent compared with the saline group.

Arg0-Ang-(1-7) at a dose of 0.5 nmol/kg, had a strong and sustained antihypertensive effect, which lasted for at least 5 hours, when compared with the saline group. The antihypertensive effect of arg0-Ang-(1-7) became significant 170 minutes after administration, reducing the PAM approximately 21 ± 3 mmHg. There was no significant difference in the effect of Ang-(1-7) on the MAP at a dose of 0.5 nmol/kg, compared to the saline group.

No further change in MAP was observed when the dose increased by 5 or 10 times; on the contrary, at doses of 2.5 and 12.5 nmol/kg, acute injection arg0-Ang-(1-7) produced an antihypertensive effect of lesser magnitude, and was observed only in the last 30 minutes of the trial period when compared with the saline group. The Ang-(1-7) at doses of 2.5 and 12.5 nmol/kg produced a significant anti-hypertensive effect after 110 minutes of the administration, when compared to the saline group; but its effect was more potent at the dose of 2.5 nmol/kg.

Acute intravenous administration of arg0-Ang-(1-7) and Ang-(1-7) at a dose of 0.1 nmol/kg, did not significantly alter PAM in SHR rats.

In relation to CF, no change was observed after the administration in bolus arg0-Ang-(1-7) and Ang-(1-7) in SHR rats, with the exception of the dose 12.5 nmol/kg Ang-(1-7), which significantly reduced the FC in the last 30 minutes of the experiment compared to the control group.

In Wistar rats (Figures 7C-1 and 7C-2) arg0-Ang-(1-7) and Ang-(1-7), at a dose of 0.5 nmol/kg, failed to induce significant changes in MAP and HR compared to the control group.

### b) acute oral administration

After 90 minutes of stabilization of cardiovascular parameters, a single dose of the peptides arg0-Ang-(1-7) and Ang-(1-7) was administered orally (gavage) in a final volume of 500 uL, to SHR and Wistar rats. The peptides were provided in hydroxypropyl beta-cyclodextrin (HPβCD) to prevent degradation from the gastrointestinal tract.

The dose was 10 µg/kg and cardiovascular parameters were continuously monitored for a period of 5 hours, with conscious animals. The values of the MAP and HR were obtained every 10 minutes throughout the experimental period.

Results are presented as mean ± standard error of the mean. Two-way analysis of variance (ANOVA) with Bonferroni post-test for multiple comparisons was used to compare the time-response curve obtained during the administration in bolus of Ang-arg0-(1-7) and Ang-(1-7). All statistical analyses were considered significant when p <0.05.

Figures 8A-1 to 8B-2 illustrate the changes in MAP and HR after acute oral administration of 10ug/kg HPβCD/arg0-Ang-(1-7) and HPβCD/Ang-(1-7) , in both Wistar and SHR rats. As can be seen in Figure 8A-1, the oral administration of arg0-Ang-(1-7) in SHR rats induced a time-dependent anti-hypertensive effect when compared with the saline group. The reduction in MAP was significant after 220 minutes from the administration of arg0-Ang-(1-7) and remained reduced until the end of the trial period. The peptide Ang-(1-7) did not induce changes in SHR at a dose of 10ug MBP/kg compared with the control group. Regarding FC (Figure 8B-1), there was no significant change after the administration of peptides.

Acute oral administration to Wistar rats of 10ug/kg HPβCD/arg0-Ang-(1-7) and the HPβCD/Ang-(1-7) did not induce significant changes in MAP (Figure 8A-2) and HR (Figure 8B-2) compared to the control group.

### Example 8 - Activation of AKT Protein by arg0-Ang-(1-7).

### a) Cardiomyocyte test cells

Wistar neonatal rat cardiomyocytes were cultured in regular growth medium consisting of DEMEM medium supplemented with 10% horse serum, 5% fetal bovine serum, 1% penicillin/streptomycin and 0.1% fibroblast inhibitor. The cells were maintained in serum free medium for three hours before the start of treatment. The study was carried out acutely, for five minutes using a concentration of 10⁻⁹ mol/L of the following drugs diluted in culture medium: Ang-(1-7), arg0-Ang-(1-7) and insulin. Cells incubated with culture medium only were used as control group.

Total protein was isolated from cardiomyocytes cells with lysis buffer containing protease inhibitors cocktail (Days-Peixoto et al., Hypertension 52: 542-548, 2008). Sixty micrograms of total protein was separated by electrophoresis, transferred to a nitrocellulose membrane, blocked with 5% skimmed milk powder diluted in Tris-saline containing 0.1% Tween, and finally incubated overnight with anti-AKT polyclonal antibody (1: 1000), anti- Ser473 phospho-Akt (1: 600), anti-glyceraldehyde-3-phosphate dehydrogenase (GAPDH - 1: 1000). Subsequently, the membranes were incubated with fluorescently labeled secondary antibody (1: 10000), followed by identification and quantitation by the Odyssey program (LI-COR).

The results are presented as mean + E.PM. The student t test was used to analyze the phosphorylation of protein kinase B (AKT) in the presence of arg0-Ang-(1-7), Ang-(1-7) and insulin, compared to the control group (*) and to compare the arg0 stimulation produced by peptide-Ang-(1-7) and Ang-(1-7) (#). All statistical analyses were considered significant when p <0.05.

The peptides arg0-Ang-(1-7) and Ang-(1-7) triggered the activation of protein kinase B (AKT) in cardiomyocyte cells, with stronger results in the presence of arg0-Ang-(1- 7) (Figure 9A).

### b) Primary pre-adipocyte cell culture

Primary cultures of pre-adipocytes extracted from epididymal adipose tissue from adult male Wistar rats was performed. The cells were grown in regular growth medium consisting of DEMEM medium supplemented with 10% fetal bovine serum and 1% penicillin/streptomycin. The preadipocytes were maintained in serum free medium for six hours before the start of treatment, which was carried out acutely, for five minutes at a concentration of 10⁻⁹ mol/L of the following drugs diluted in culture medium: arg0-Ang-(1-7); insulin; Arg0-Ang-(1-7) + insulin; and arg0-Ang-(1-7) + A-779. MAS receptor antagonist, A-779, was added at a concentration of 10⁻⁸ mol/L 10 minutes before stimulation with the peptide arg0-Ang-(1-7). Cells incubated with culture medium alone were used as control group.

Total protein was isolated from the pre-adipocytes with lysis buffer containing a cocktail of protease inhibitors from a commercial ELISA kit. Next, the extracted proteins were measured by the Bradford method (Bradford, MM, Analytical Biochemistry 72: 248-254, 1976) and used for quantitation of the active form of AKT using a commercial ELISA kit according to the manufacturer's specification.

The results are presented as mean + SEM. The student t test was used to analyze the phosphorylation of serine 473 residue of AKT in the presence of arg0-Ang-(1-7); insulin; Arg0-Ang-(1-7) + insulin; and arg0-Ang-(1-7) + A-779 compared to the control group (*). All statistical analyses were considered significant when P <0.05.

The peptide arg0-Ang-(1-7) increased activation of AKT in pre-adipocyte cells, and this result is blocked in the presence of the MAS receptor antagonist, A-779 (Figure 9B). Therefore, the arg0-Ang-(1-7) is a peptide that activates the insulin signaling pathway, increasing sensitivity to the actions of this hormone.

### Example 9 - Effect of arg0-Ang-(1-7) Peptide in Treatment in a Metabolic Syndrome Model.

Because the peptide arg0-Ang-(1-7) is able to activate the insulin signaling pathway, the effect of treatment with arg0-Ang-(1-7) was evaluated in a metabolic syndrome model.

Metabolic syndrome was induced in Sprague-Dawley rats by ingestion of 10% fructose in water for 9 weeks. In the last 4 weeks of the experimental protocol, the animals were orally treated (10 ug/kg body weight) with arg0-Ang-(1-7) with β-Hydroxy-propylcyclodextrin. The animals were divided into 4 experimental groups: vehicle control - no intake of fructose and treated only with the inclusion compound; Arg0 control - without fructose intake and treated with arg0-Ang-(1-7); Vehicle Fructose - fructose intake and treated only with the inclusion compound; arg0 and Fructose - fructose intake and treated with arg0-Ang-(1-7).

In this experimental protocol, we assessed body weight gain; visceral adiposity; serum and hepatic levels of triacylglycerol and cholesterol; glucose tolerance and insulin resistance.

In general, treatment with arg0-Ang-(1-7) normalized all metabolic changes evaluated in the metabolic syndrome model induced by intake of a high concentration of fructose. The main metabolic benefits promoted by treatment with arg0-Ang-(1-7) including: improved insulin sensitivity and glucose tolerance; decrease of body weight gain and body fat mass; decreased levels of serum and liver triacylglycerol in addition to the reduction of serum and hepatic cholesterol levels produced in both treated groups.

### a) measuring body weight gain and visceral adiposity in treaty metabolic syndrome model arg0-angiotensin-(1-7)

Body weight gain was evaluated in the 8th week of the experimental protocol. At the end of the experiments, the epididymal and mesenteric adipose tissue was removed, weighed and corrected for total body weight to assess visceral adiposity.

Results are presented as mean ± SEM. Two-away analysis of variance (ANOVA) with Bonferroni's multiple comparison post-test was used to compare the gain of body weight and visceral adiposity between groups. All statistical analyses were considered significant when p <0.05.

Treatment with arg0-Ang-(1-7) lessened the body weight gain (A) and blocked the increase in mass of the epididymal adipose tissue (B) and mesenteric adipose tissue (C) induced by the intake of fructose (Figure 10). Therefore, the arg0-Ang-(1-7) decreased body weight gain and prevented the development of metabolic syndrome, visceral adiposity in model induced by fructose.

### b) Evaluation of serum and hepatic levels of triacylglycerol and cholesterol in a metabolic syndrome model treated with arg0-angiotensin-(1-7)

At the end of the experimental protocol, the serum and liver of the animals were collected for biochemical evaluation of lipid levels, which were measured using commercial kits according to the manufacturer's specifications.

Results are presented as mean ± SEM. Two-away analysis of variance (ANOVA) with post-test Bonferroni multiple comparison or the Student t test was used to compare serum and hepatic levels of triacylglycerol and cholesterol between groups. All statistical analyses were considered significant when p <0.05.

Treatment with arg0-Ang-(1-7) blocked the increase in triglyceride levels promoted by fructose intake both in serum (A) and in the liver (B) of the animals (Figure 11). In addition, treatment with arg0-Ang-(1-7) reduced serum (C) and liver (d) cholesterol levels in both treated groups (Figure 11). Therefore, the arg0-Ang-(1-7) improved the lipid profile in both the metabolic syndrome model and in normal animals.

### c) Evaluation of glucose tolerance and insulin resistance in a metabolic syndrome model treated with arg0-angiotensin-(1-7).

A glucose tolerance test was performed by evaluation of basal glycemia and 15, 30, 45, 60, 90 and 120 minutes after the oral administration of glucose (2 g/kg body weight). Basal insulin release also was evaluated during the period of 15 and 120 minutes of the experimental protocol. The blood glucose levels and fasting insulin (6-8 hours) were used to estimate the degree of insulin resistance using the Homeostatics Model Assessment Insulin Resistance calculation (HOMA-IR; Matthews et al, Diabetologia 28: 412 - 419, 1985).

Results are presented as mean ± SEM. Two-away analysis of variance (ANOVA) with Bonferroni's multiple comparison post-test was used to compare the restoration of blood glucose, insulin release and the degree of insulin resistance between groups. All statistical analyses were considered significant when p <0.05.

Treatment with arg0-Ang-(1-7) normalized the restoration of normoglycemia (A and B) as well as the release of insulin (C) compensating for the oral administration of glucose, changed by fructose ingestion (Figure 12). In addition, arg0-Ang-(1-7) reduced the high ratio between insulin and fasting glucose (D) presented by the group ssubjected to fructose consumption (Figure 12). Therefore, the arg0-Ang-(1-7) normalized glucose intolerance and insulin resistance in a metabolic syndrome model.

### Example 10 - Anti-proliferative Effect of arg0-Ang-(1-7).

Human alveolar adenocarcinoma cells (A549), cultured in DEMEM supplemented with 10% fetal bovine serum and 1% penicillin/streptomycin, were distributed into 96 wells plate at the density of 2 x 10⁴ cells/well. After 48 hours of incubation in an incubator at 37 ° C, the arg0-angiotensin-(1-7) and angiotensin (1-7) peptides were added to the wells and serially diluted (1x10⁻⁴ to 2x10⁻⁷M) in culture medium free of fetal bovine serum. Wells not plated with cells and wells with cells but stimulated only with culture medium were used as controls. The stimulus, which lasted 48 hours, was followed by the addition of AlamarBlue reagent (Invitrogen), used to detect cell viability.

The results show that the arg0-angiotensin-(1-7) showed an anti-proliferative effect similar to that of angiotensin-(1-7) (Figure 13).

### Example 11 - Arg-Arg-Ang-(1-7) Relaxation Effect in Wistar Rat Aortic Rings.

Aorta rings of Wistar rat containing functional endothelium were pre-contracted with Phe and during the tonic phase of the contraction, a cumulative concentration-response curve was constructed with Arg-Arg- Ang-(1-7) (10⁻¹² to 10⁻⁶ M), and arg0-Ang-(1-7) was used as positive control.

Results are presented as mean ± standard error of the mean. Two-way analysis of variance (ANOVA) with Bonferroni's multiple comparison post-test was used to compare the concentration response curves obtained in the aortic rings. The vasodilator effect of Arg-Arg-Ang (1-7) was expressed as the percentage of decrease in peak concentration induced by phenylephrine. All statistical analyses were considered significant when p <0.05.

As can be seen in Figure 14, Arg-Arg peptide Ang-(1-7) produced a vasodilator effect similar to arg0-Ang-(1-7) in Wistar rat aortic rings. The result shows that the addition of a further amino terminal arginine at position Ang-(1-7) did not result in increased vasodilatory effect, at least in rats.

## Claims

1. Peptide **characterized in that** (arg0) n-angiotensin- (1-7) where n 1 to 1, and presenting the amino acid sequence shown in SEQ ID No. 1, where Xaa represents a variation 1-1 L-Arg amino acids.

2. A pharmaceutical composition comprising the peptide (Arg0) n-angiotensin- (1-7) represented by SEQ ID No. 1 and described in claim 1 and pharmaceutically acceptable excipients.

3. A pharmaceutical composition according to claim 2, comprising the peptide (arg0) n-angiotensin- (1-7) represented by SEQ ID NO: 1, optionally associated with a system carrier or controlled drug delivery system.

4. A pharmaceutical composition according to claim 3, **characterized by** the carrier system or delivery system controlled is selected from the group comprising cyclodextrins, polymers, mucoadhesive polymers, lipidic vesicles, liposomes, polimersomas, SOLID lipid nanoparticles, polymeric micro- and nanoparticles, micro- and nanocapsules, micro- and nanoemulsions, dendrimers, micelles, polymeric micelles, inorganic nanoparticles, carbon nanoparticles, transdermal patches, implantable polymeric matrices or devices that may be deployed.

5. A pharmaceutical composition according to claims 2 to 4, **characterized by** being administered by oral, intramuscular, intravenous, subcutaneous, topical, transdermal, nasal, pulmonary, inhalation, for devices that can be implanted or injected, preferably, orally.

6. Use of the peptide wherein the peptide is that described in claim 1 in the preparation of medicaments for treatment diseases or disorders of the genito-urinary tract, cardiopulmonary and involving the liver, metabolic, neural, musculoskeletal, renal, cutaneous, carcinogenic, alopecia or vascular disorders.

7. Use of the pharmaceutical composition being **characterized in that** described in claims 2 to 5 in the treatment of diseases or disorders genito-urinary tract, cardiopulmonary and involving the liver, metabolic, neural, musculoskeletal, renal, cutaneous, carcinogenic, alopecia or vascular disorders.
